Europäisches Patentamt

European Patent Office

Office européen des brevets

(11) Veröffentlichungsnummer: **0 360 959**
**A1**

# (12) EUROPÄISCHE PATENTANMELDUNG

(21) Anmeldenummer: 89105423.1

(22) Anmeldetag: 28.03.89

(51) Int. Cl.⁵: **A61B 1/24**

(30) Priorität: 28.09.88

(43) Veröffentlichungstag der Anmeldung:
04.04.90 Patentblatt 90/14

(84) Benannte Vertragsstaaten:
CH DE ES FR IT LI

(71) Anmelder: **Kulzer GmbH**
**Philipp-Reis-Strasse 8**
**D-6393 Wehrheim (TS.)1(DE)**

(72) Erfinder: **Schödel, Dieter, Dr.**
**Abeggstrasse 5**
**D-6200 Wiesbaden(DE)**
Erfinder: **Oppawsky, Steffen**
**Gartenfeldstrasse 22a**
**D-6380 Bad Homburg(DE)**
Erfinder: **Grimm, Ekkehard**
**Blumenstrasse 44**
**D-6050 Offenbach(DE)**

(74) Vertreter: **Heinen, Gerhard, Dr.**
**Heraeus Holding GmbH Heraeusstrasse**
**12-14**
**D-6450 Hanau am Main(DE)**

(54) Lichtleiter für ein Lichtgerät für dentale Zwecke.

(57) Es ist ein Lichtleiter für ein Lichtgerät für dentale Zwecke zur Behandlung im Mund bekannt mit einem mittels Halterung (4) vor dem Lichtaustrittsende (12) des Lichtleiters (1) angeordneten, das austretende Licht umlenkenden Spiegel (17). Um einen solchen Lichtleiter derart auszugestalten, daß einerseits die zu behandelnden Bereiche der Zähne gezielt bestrahlt werden können, der für den Anwender leicht handhabbar ist und gleichzeitig so aufgebaut ist, daß er dem Anwender einen guten Einblick auf die zu bestrahlende Stelle ermöglicht, wird die Halterung als gegenüber dem Querschnitt des Lichtleiters dünner stabförmiger Träger (5) ausgebildet, der mit einem Randbereich des Spiegels derart verbunden ist, daß er den Abstrahlbereich des vom Spiegel reflektierten Austrittsstrahles nicht durchsetzt, und weist der Spiegel an seiner breitesten Stelle einen Durchmesser auf, der mindestens dem doppelten Durchmesser des Lichtleiter-Austrittsendes senkrecht zur Achse des Lichtleiters entspricht.

FIG.2

## Lichtleiter für ein Lichtgerät für dentale Zwecke

Die vorliegende Erfindung betrifft einen Lichtleiter für ein Lichtgerät für dentale Zwecke zur Behandlung im Mund mit einem mittels Halterung vor dem Lichtaustrittsende des Lichtleiters angeordneten, das austretende Licht umlenkenden Spiegel.

Ein solcher Lichtleiter ist aus der DE-OS 23 08 554 bekannt, die ein Zahnbehandlungsgerät zur Kariesprophylaxe betrifft. Der dort beschriebene Lichtleiter wird in Verbindung mit einem Laser eingesetzt und ist am Ende eines Gelenkarmes oder direkt ausgangsseitig des Lasers angeordnet. Eingangsseitig des Lichtleiters befindet sich eine Fokussierungslinse; ausgangsseitig ist eine Hülse aufgesetzt, die am Ende ein Prisma oder einen Spiegel trägt, um den aus dem Lichtleiterende austretenden Strahl um 90° umzulenken. Durch diese Anordnung soll der im Mund zu bestrahlende Bereich der Zähne bzw. Bereiche des Zahnfleisches gut zugänglich sein.

Der vorliegenden Erfindung liegt nun die Aufgabe zugrunde, einen Lichtleiter für ein Gerät für dentale Zwecke zur Behandlung im Mund, insbesondere zur Aushärtung von Dentalmaterialien im Mund, zu schaffen, mit dem einerseits die zu behandelnden Bereiche der Zähne gezielt bestrahlt werden können, der für den Anwender leicht handhabbar ist und gleichzeitig so aufgebaut ist, daß er dem Anwender einen guten Einblick auf die zu bestrahlende Stelle ermöglicht.

Gelöst wird die Aufgabe bei einem Lichtleiter der eingangs beschriebenen Art dadurch, daß die Halterung als gegenüber dem Querschnitt des Lichtleiters dünner stabförmiger Träger ausgebildet ist, der mit einem Randbereich des Spiegels derart verbunden ist, daß er den Abstrahlbereich des vom Spiegel reflektierten Austrittsstrahles nicht durchsetzt, und daß der Spiegel an seiner breitesten Stelle einen Durchmesser aufweist, der mindestens dem doppelten Durchmesser des Lichtleiter-Austrittsendes senkrecht zur Achse des Lichtleiters entspricht. Der vor dem Austrittsende des Lichtleiters angeordnete Spiegel dient sowohl zur Umlenkung des aus dem Lichtleiter austretenden Lichtstrahles als auch als Beobachtungsspiegel für den Benutzer dieses Lichtleiters, beispielsweise für den behandelnden Zahnarzt. Da die den Lichtleiter handhabende Person den Spiegel aus einer anderen Richtung einsieht als die Richtung des auf den Spiegel auftreffenden Lichtstrahles, wird die Person durch das vom Spiegel reflektierte Licht nicht geblendet. Der stabförmige Träger oder Arm, der am Ende des Lichtleiters befestigt den Spiegel trägt, ist sehr dünn ausgebildet, so daß er die Spiegelfläche praktisch nicht verdeckt und damit die Einsicht auf den Spiegel nicht behindert. Bevorzugt ist dieser Arm im oberen Bereich am Rand des Spiegels befestigt, so daß der Beobachter seitlich in den Spiegel einsehen kann. Die zu behandelnden Stellen eines Zahnes können mit diesem Lichtleiter gezielt bestrahlt werden, da sie über den Spiegel gut eingesehen werden können. In dieser Anordnung ist der Lichtleiter mit einer Hand bedienbar; ein gesonderter Spiegel zur Beobachtung der zu bestrahlenden Stellen ist nicht erforderlich. Um ein ausreichend großes Beobachtungsfeld zu bieten, sollte der Spiegel an seiner breitesten Stelle etwa dem doppelten Durchmesser des Lichtleiters entsprechen.

Üblicherweise zeigen die aus dem Ende eines Lichtleiters austretenden Strahlen eine Divergenz von einigen Grad, d. h. die austretende Strahlung beschreibt einen sich ausweitenden Kegel. Um die Strahlung wieder zu bündeln bzw. zu fokussieren, wird bevorzugt als Spiegel ein Hohlspiegel eingesetzt. Um den Strahlquerschnitt ausgangsseitig des Umlenk-Spiegels zu verändern, ist der Spiegel mittels des Trägers in Richtung der Lichtleiterachse verschiebbar angeordnet. Mit sich vergrößerndem Abstand des Spiegels zu dem Lichtleiter-Austrittsende wird - infolge der Divergenz des austretenden Lichtstrahles - der Strahlquerschnitt vergrößert, während mit sich verringerndem Abstand zu dem Lichtleiter-Austrittsende der Strahlquerschnitt kleiner wird.

In vorteilhaften Ausbildungen kann der Träger mittels eines Klemmteiles, das den Lichtleiter zumindest teilweise umgreift, am Außenmantel des Lichtleiters festgeklemmt werden. Eine solche Klemmeinrichtung bietet gleichzeitig die Möglichkeit, den Träger mit dem Spiegel in Richtung der Lichtleiter-Achse zu verschieben, ohne daß hierzu gesonderte Bauteile erforderlich wären. Eine weitere sehr einfache Möglichkeit, den Träger am Lichtleiter zu befestigen, bietet eine Magnetkupplung, die beispielsweise in Form von ringförmigen auf den Lichtleiter auf geschobenen Elementen gebildet werden kann. Durch Zwischenringe zwischen dem am Lichtleiter befestigten Kupplungsteil und dem Kupplungsteil des Trägers kann in einer solchen Verbindung der Abstand des Spiegels zum Lichtleiteraustrittsende variiert werden. Um den Spiegel in seinem Winkel zur Achse des Lichtleiters einstellen zu können, sollte der Spiegel schwenkbar an dem Träger angeordnet sein. Bevorzugt schneidet die Spiegelebene die Achse des Lichtleiters unter einem Winkel zwischen 90° und 30°. Um den Spiegel in den verschiedenen Winkelstellungen zu fixieren, sind Rastelemente von Vorteil.

Eine weitere, sehr vorteilhafte Möglichkeit zur Einstellung des Spiegels kann dadurch erzielt werden, daß der Träger wenigstens teilweise aus leicht biegbarem Material besteht.

Bevorzugt wird der Durchmesser des Spiegels, ausgehend von einem kreisrunden Spiegel, von 15 mm bis 25 mm gewählt, wobei üblicherweise der Lichtleiter einen Durchmesser von etwa 5 mm bis 7 mm aufweist. In Achsrichtung des Lichtleiters gesehen sollte der Abstand des Befestigungspunktes des Spiegels an dem Träger zwischen 5 mm und 60 mm vom Lichtleiter-Austrittsende betragen, wobei der bevorzugte Bereich zwischen 5 mm und etwa 15 mm liegt.

Um mit dem Lichtleiter bzw. dem Spiegel möglichst nahe an den zu bestrahlenden Bereich zu gelangen, ist der Randbereich des Spiegels, der vom Austrittsende des Lichtleiters am weitesten entfernt ist, quer abgeflacht verlaufend ausgebildet. Als Orientierungshilfe für den Anwender des Lichtleiters, welcher Bereich des Zahnes bestrahlt wird, haben sich Markierungs- oder Orientierungshilfen auf der Spiegelfläche als vorteilhaft erwiesen, beispielsweise durch einen Markierungsring, der in die Spiegelfläche eingekratzt oder auf die Spiegelfläche aufgedruckt ist.

Gerade zur Aushärtung von Dentalmaterialien im Mund hat sich der erfindungsgemäße Lichtleiter mit dem vorgesetzten Umlenk- und Beobachtungsspiegel als vorteilhaft erwiesen.

Ausführungsbeispiele der Erfindung werden nachfolgend anhand der Zeichnungen näher erläutert. In der Zeichnung zeigt

Figur 1 eine perspektivische Ansicht eines Lichtleiters mit darauf aufgesetztem Umlenkspiegel,

Figur 2 einen Längsschnitt entlang der Achse des Lichtleiters durch eine Anordnung wie sie die Figur 1 zeigt,

Figur 3 eine zu den Anordnungen nach den Figuren 1 und 2 alternative Befestigung des Spiegels am Lichtleiter,

Figur 4 eine Schwenkanordnung des Spiegels an dem Träger und

Figur 5 eine Ansicht auf den Spiegel in Richtung des Pfeiles V in Figur 3.

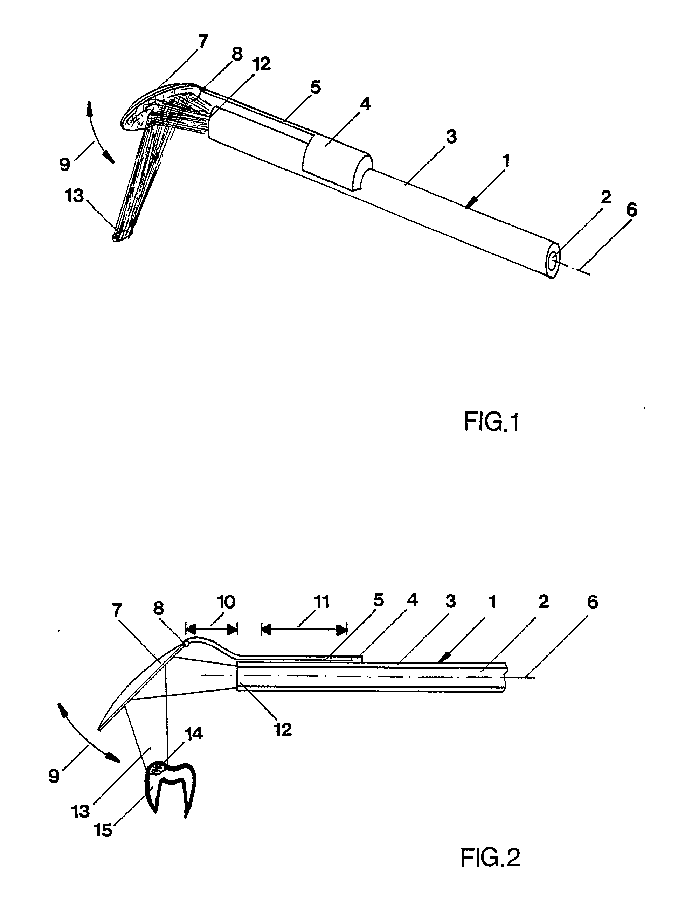

Die Figuren 1 und 2 zeigen das Ende eines Lichtleiters 1, der von einem Außenmantel 3 umgeben ist. An dem Lichtleiter 1 ist mit einem Klemmteil 4 ein stabförmiger Träger 5, der im wesentlichen in Richtung der Achse 6 des Lichtleiters verläuft, befestigt. Am Ende dieses stabförmigen Trägers, der in der Ausführungsform nach der Figur 2 von der Achse 6 des Lichtleiters aus gesehen an seinem Ende geringfügig nach oben verlaufend gebogen ist, ist ein Spiegel 7, bei dem es sich um einen Hohlspiegel handelt, über ein Gelenk 8 befestigt. Durch das Gelenk 8, das eine Schwenkachse quer zur Achse 6 des Lichtleiters 1 bildet, ist der Spiegel 7 in Richtung des Schwenkpfeiles 9 einstellbar. Der Spiegel 7 mit kreisrunder Außenkontur besitzt etwa den doppelten Durchmesser des Lichtleiters 1, so daß - wie die Figur 1 zeigt - auf dem hinteren Ende des Lichtleiters, d. h. aus Richtung einer den Lichtleiter handhabenden Person gesehen, eine ausreichende Spiegelfläche verbleibt, die nicht durch den Lichtleiter verdeckt wird; dies gilt insbesondere dann, wenn der Abstand des Befestigungspunktes oder Gelenkes 8, durch die Abstandspfeile 10 angedeutet, sehr gering ist, beispielsweise entsprechend dem gezeigten Beispiel von 10 mm. Mittels des Klemmteiles 4, mit dem der Träger zusammen mit dem Spiegel an dem Lichtleiter festgeklemmt ist, kann der Spiegel um den Abstand 11, der etwa 20 mm beträgt, wie aus Figur 2 ersichtlich ist, in Richtung der Achse 6 des Lichtleiters 1 verschoben werden. Falls das Klemmteil 4 zu dem Austrittsende 12 des Lichtleiters hin verschoben wird, vergrößert sich der Abstand 10 zwischen dem Lichtleiteraustrittsende 12 und dem Gelenk 8, wodurch infolge des divergierenden auf den Spiegel 7 auftreffenden Strahles das von dem Spiegel 7 reflektierte Strahlenbündel 13 vergrößert wird. Durch das Verschieben des Klemmteiles 4 kann der Strahlquerschnitt entsprechend dem zu bestrahlenden Bereich 14 eines Zahnes 15 eingestellt werden. Der Lichtleiter 1 kann mit einer Hand bedient werden, wobei vom Anwender der zu bestrahlende Bereich, insbesondere dann, wenn ein im Mund befindlicher Zahn 15 bestrahlt werden soll, über den Spiegel 7 beobachtbar ist.

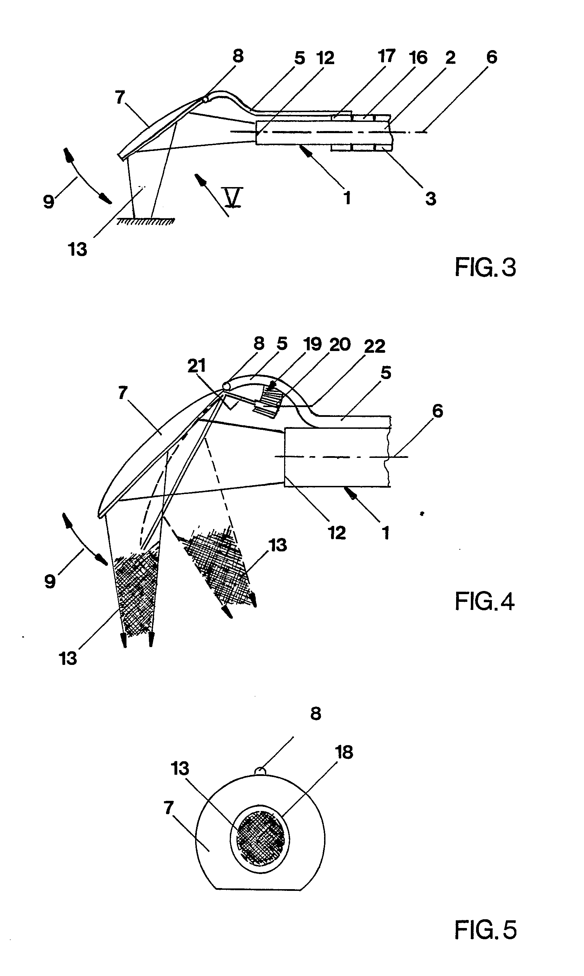

Figur 3 zeigt eine Magnetkupplung, bestehend aus einem über das Faserbündel 2 des Lichtleiters 1 geschobenen ersten Kupplungsteil 16 und einem zweiten Kupplungsteil 17, an dem der stabförmige Träger 5 befestigt ist. Um den Abstand 10 zwischen dem Austrittsende 12 und dem Gelenk 8 des Spiegels 7 zu verändern, können zwischen das erste Kupplungsteil 16 und das zweite Kupplungsteil 17 nicht näher dargestellte Zwischenringe eingeschoben werden.

Der Spiegel 7 nach Figur 3 und Figur 5 mit kreisrunder Außenkontur ist an seinem unteren Rand, der vom Austrittsende 12 des Lichtleiters 1 am weitesten entfernt ist, quer abgeflacht, so daß der Spiegel 7 sehr nahe an das zu bestrahlende Objekt gebracht werden kann.

Um dem Anwender die Orientierung zu erleichtern, können auf der Spiegelfläche des Spiegeles 7 entsprechend Figur 4 Markierungen 18, die den Auftreffbereich des Strahlenbündels bzw. den Reflexionsbereich auf der Spiegelfläche umschließen, vorgesehen sein. Solche Markierungen 18 können beispielsweise in die Spiegelfläche eingeritzt oder aber durch Farbe auf die Spiegeloberfläche aufgebracht werden.

Um den Spiegel 7 zu der Achse 6 des Lichtleiters 1 einstellen bzw. feststellen zu können, ist im Bereich des Gelenkes 8 ein Rastelement 19 mit rillen 20 vorgesehen in Form eines Steges 21 an dem Träger 5, in den eine Rastnase 22, die fest mit dem Spiegel 7 verbunden ist, einrastet. Durch dieses Rastelement 19 ist ein einfaches Verschwenken des Spiegels 7 in Richtung des Schwenkpfeiles 9 möglich; in Figur 4 sind zwei verschiedene Raststellungen gezeigt.

**Ansprüche**

1. Lichtleiter für ein Lichtgerät für dentale Zwecke zur Behandlung im Mund mit einem mittels Halterung vor dem Lichtaustrittsende des Lichtleiters angeordneten, das austretende Licht umlenkenden Spiegel, dadurch gekennzeichnet, daß die Halterung (4, 5; 16, 17, 5) als gegenüber dem Querschnitt des Lichtleiters (1) dünner stabförmiger Träger (5) ausgebildet ist, der mit einem Randbereich des Spiegels (7) derart verbunden ist, daß er den Abstrahlbereich des vom Spiegel (7) reflektierten Austrittsstrahles nicht durchsetzt, und daß der Spiegel (7) an seiner breitesten Stelle einen Durchmesser aufweist, der mindestens dem doppelten Durchmesser des Lichtleiter-Austrittsendes (12) senkrecht zur Achse (6) des Lichtleiters (1) entspricht.

2. Lichtleiter nach Anspruch 1, dadurch gekennzeichnet, daß der Spiegel (7) ein Hohlspiegel ist.

3. Lichtleiter nach Anspruch 1 oder 2, dadurch gekennzeichnet, daß der Träger (5) am Lichtleiter (1) in Richtung zu der Lichtleiter-Achse (6) verschiebbar angeordnet ist.

4. Lichtleiter nach einem der Ansprüche 1 bis 3, dadurch gekennzeichnet, daß der Träger (5) mittels Magnetkupplung (16, 17) am Lichtleiter (1) befestigt ist.

5. Lichtleiter nach einem der Ansprüche 1 bis 4, dadurch gekennzeichnet, daß der Träger (5) mittels eines Klemmteiles (4), das den Lichtleiter (1) zumindest teilweise umgreift, festgeklemmt ist.

6. Lichtleiter nach einem der Ansprüche 1 bis 5, dadurch gekennzeichnet, daß der Spiegel (7) schwenkbar an dem Träger (5) angeordnet ist.

7. Lichtleiter nach Anspruch 6, dadurch gekennzeichnet, daß der Spiegel (7) mittels Rastelementen (19) in seiner Schwenkstellung feststellbar ist.

8. Lichtleiter nach einem der Ansprüche 1 bis 7, dadurch gekennzeichnet, daß der Träger (5) wenigstens teilweise aus biegbarem Material besteht.

9. Lichtleiter nach einem der Ansprüche 1 bis 8, dadurch gekennzeichnet, daß der Spiegel (7) einen Durchmesser von 15 mm bis 25 mm aufweist.

10. Lichtleiter nach einem der Ansprüche 1 bis 9, dadurch gekennzeichnet, daß der Abstand (10) des Befestigungspunktes (8) des Spiegels (7) an dem Träger (5) in Richtung der Achse (6) des Lichtleiters (1) gesehen zwischen 5 mm und 60 mm vom Lichtleiter-Austrittsende (12) beträgt.

11. Lichtleiter nach einem der Ansprüche 1 bis 10, dadurch gekennzeichnet, daß der Randbereich des Spiegels, der vom Austrittsende (12) des Lichtleiters (1) am weitesten entfernt ist, quer abgeflacht verläuft.

12. Lichtleiter nach einem der Ansprüche 1 bis 11, dadurch gekennzeichnet, daß der Spiegel (7) auf der Spiegelfläche Markierungs- oder Orientierungshilfen (18) aufweist.

13. Verwendung des Lichtleiters nach einem der Ansprüche 1 bis 12 für ein Lichtgerät zur Aushärtung von Dentalmaterialien im Mund.

FIG.1

FIG.2

FIG.3

FIG.4

FIG.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-3 638 013 (R. F. KELLER) <br> * Spalte 2, Zeile 40 - Spalte 6, Zeile 52; Figuren 1-10 * | 1 | A 61 B 1/24 |
| Y | | 2 | |
| A | | 3,5,8, 11 | |
| | --- | | |
| X | EP-A-0 122 537 (SUMITOMO ELECTRIC INDUSTRIES LTD.) <br> * Seite 7, Zeile 19 - Seite 13; Zeile 4; Figuren 4-10 * | 1 | |
| Y | | 2 | |
| | --- | | |
| Y | FR-A- 740 486 (H. PAPE) <br> * Seite 2, Zeilen 27-35; Figur 4 * | 2 | |
| | --- | | |
| A | US-A-4 629 425 (S. G. DETSCH) <br> * Spalte 5, Zeile 27 - Spalte 6, Zeile 68; Figuren 5-10 * | 1,3,5 | |
| | ----- | | |

| RECHERCHIERTE SACHGEBIETE (Int. Cl.5) |
|---|
| A 61 B 1/00 <br> G 02 B 23/00 |

Der vorliegende Recherchenbericht wurde für alle Patentansprüche erstellt

| Recherchenort | Abschlußdatum der Recherche | Prüfer |
|---|---|---|
| BERLIN | 13-11-1989 | WEIHS J.A. |